# EUROPEAN PATENT APPLICATION

(11) **EP 1 760 077 A1**
(43) Date of publication of application: **07.03.2007**
(21) Application number: 05112284.4
(22) Date of filing: 31.01.2005
(51) Int. Cl.: C07D 215/18

(54) **Montelukast free acid polymorphs**

(30) Priority: 30.01.2004 US 540840; 22.06.2004 US 582237
(62) Divisional of application: 05712362.2
(71) Applicant: TEVA PHARMACEUTICAL INDUSTRIES LTD., 49131 Petah Tiqva (IL)
(72) Inventor: Niddam-Hildesheim, Valerie, 40696, Ein Vered (IL); Aronhime, Judith, 76217, Rehovot (IL); Chen, Kobi, 47291, Ramat HaSharon (IL)
(74) Representative: Nachshen, Neil Jacob

(57) **Abstract**

The present invention relates to amorphous and polymorphic forms of montelukast free acid.

## Description

This application claims the benefit of U.S. Provisional Patent Applications Ser. Nos. 60/540,840 filed January 30, 2004 and 60/582,237 filed June 22, 2004.

### FIELD OF THE INVENTION

The present invention relates to the solid state chemistry of montelukast free acid.

### BACKGROUND OF THE INVENTION

Montelukast is a selective, orally active leukotriene receptor antagonist that inhibits the cysteinyl leukotriene CysLT₁ receptor. Leukotrienes are associated with the inflammation and constriction of airway muscles and the accumulation of fluid in the lungs. Montelukast sodium is a useful therapeutic agent for treating respiratory diseases such as asthma and allergic rhinitis.

The chemical name for montelukast sodium is: [*R*-(*E*)]-1-[[[1-[3-[2-(7-chloro-2-quinolinyl)ethenyl]phenyl]-3-[2-(1-hydroxy-1-methylethyl)phenyl]propyl]thio]methyl]cyclopropaneacetic acid, monosodium salt. Montelukast sodium is a hygroscopic, optically active, white to off-white powder. Montelukast sodium is freely soluble in methanol, ethanol, and water and practically insoluble in acetonitrile.

Montelukast free acid is represented by the formula:

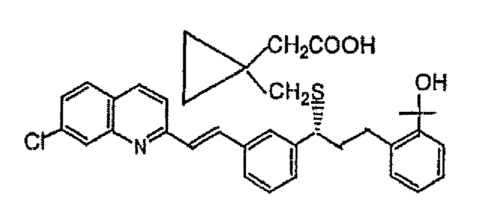

U.S. Patent Number 6,320,052 discloses that the available processes for crystallizing montelukast sodium are "not particularly suitable for large-scale production" because of the "tedious chromatographic purification" technique required and because the "product yields are low."

U.S. Patent Number 5,565,473 discloses a synthetic process for montelukast sodium, wherein the montelukast acid is converted directly to the corresponding sodium salt, without isolation of the acid The lack of montelukast free acid in solid form is problematic because it does not allow for purification of montelukast sodium Montelukast sodium often contains impurities as a result of the manufacturing process. Such impurities may be challenging to remove from the final product.

### SUMMARY OF THE INVENTION

In one embodiment, the present invention provides solid montelukast free acid. In one embodiment, the solid montelukast free acid is amorphous. In another, the solid montelukast free acid is crystalline.

In one embodiment, the present invention provides a process for preparing amorphous form montelukast free acid by dissolving montelukast salt in water to form a solution, combining an acid with the solution, maintaining the solution to obtain a precipitate, and recovering the precipitate, which is amorphous form montelukast free acid.

In another embodiment, the present invention provides a process for preparing crystalline montelukast free acid by dissolving montelukast salt in water to form a solution, maintaining the solution to obtain a precipitate, and recovering the precipitate, which is crystalline montelukast free acid.

In yet another embodiment, the present invention provides crystalline Forms I and II of montelukast free acid. The present invention also provides processes of preparing the same.

The present invention provides pharmaceutical compositions and methods of treating asthma utilizing montelukast free acid.

In another embodiment, the present invention provides a process for preparing montelukast sodium by obtaining solid montelukast free acid, crystallizing the montelukast free acid, and converting the montelukast free acid to montelukast sodium.

### BRIEF DESCRIPTION OF THE FIGURES

Figure 1 illustrates the X-ray powder diffraction pattern for montelukast free acid Form I.
Figure 2 illustrates the X-ray powder diffraction pattern for montelukast free acid Form II.

### DETAILED DESCRIPTION OF THE INVENTION

The present invention provides solid montelukast free acid. Preparation of montelukast free acid in a solid form opens a new path for purifying the sodium salt. Further, solid compounds are easier to handle. Solid compounds may allow for more convenient means of manufacturing, packaging, transporting, and administrating.

The present invention also provides processes for preparing montelukast free acid. The invention provides pharmaceutical compositions containing montelukast free acid of the invention and methods of treating respiratory diseases using the same

Another embodiment of the invention encompasses crystalline montelukast free acid. Crystalline montelukast free acid is montelukast with a free carboxylic acid group, as opposed to the sodium salt.

The present invention relates to the solid state physical properties of montelukast. These properties can be influenced by controlling the conditions under which montelukast is obtained in solid form. Solid state physical properties include, for example, the flowability of the milled solid.. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch, or tribasic calcium phosphate,

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs, and other liquid medicaments. The solid state form of a compound may also affect its behavior on compaction and its storage stability.

These practical physical characteristics are influenced by the conformation and orientation of molecules in the unit cell, which defines a particular polymorphic form of a substance. The polymorphic form may give rise to thermal behavior different from that of the amorphous material or another polymorphic form. Thermal behavior is measured in the laboratory by such techniques as capillary melting point, thermogravimetric analysis (TGA), and differential scanning calorimetry (DSC) and can be used to distinguish some polymorphic forms from others. A particular polymorphic form may also give rise to distinct spectroscopic properties that may be detectable by powder X-ray crystallography, solid state ¹³C NMR spectrometry, and infrared spectrometry.

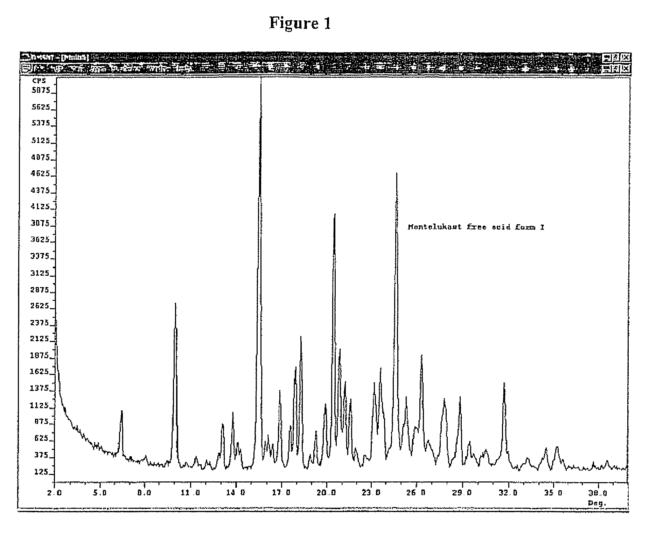

Another embodiment of the invention encompasses crystalline montelukast free acid Form I, herein defined as Form I. Form I is identified by an X-ray powder diffraction pattern with peaks at 6.5, 10.0, 13.1, 15.5, 17.6, and 18.3 degrees two-theta ± 0.2 degrees two-theta. Form I may be identified further by X-ray powder diffraction peaks at 20 4, 24.6, 26.3, 27.8, 28.8, and 31.7 degrees two-theta ± 0.2 degrees two-theta, as illustrated by Figure 1.

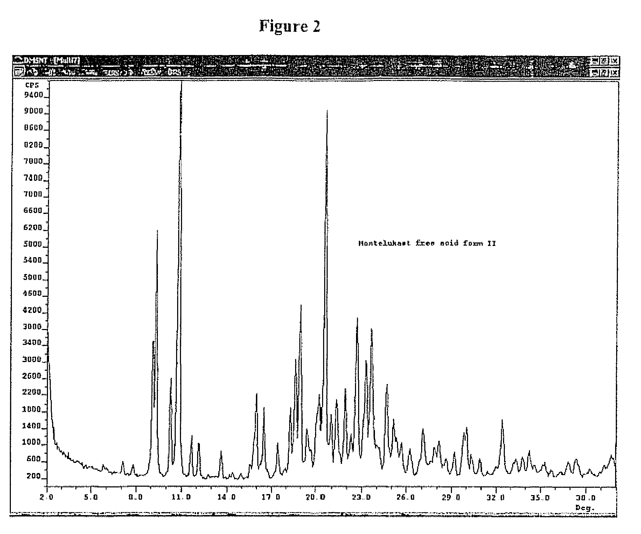

Another embodiment of the invention encompasses crystalline montelukast free acid Form II, herein defined as Form II. Form II is identified by an X-ray powder diffraction pattern with peaks at 9.1, 9.4, 10.3, 10.8, and 19.0 degrees two-theta ± 0.2 degrees two-theta. Form II may be identified further by X-ray powder diffraction peaks at 16.0, 16.5, 18.7, 20.6, 22.7, 23.2, and 23.6 degrees two-theta ± 0.2 degrees two-theta, as illustrated by Figure 2.

The invention also provides hydrates and solvates of crystalline montelukast free acid.

One embodiment of the invention encompasses processes for crystallizing montelukast free acid. In one embodiment, the process for preparing crystalline forms of montelukast free acid includes the steps of crystallizing the crystalline form from a solution of montelukast in a solvent, and recovering the crystalline form.

The solution is prepared by dissolving montelukast in a solvent. For the dissolving step, the montelukast can be any crystalline or amorphous form of montelukast, including any salts, solvates, and hydrates. The form of the montelukast for the dissolving step is unimportant because the structure will be lost in solution. The solvent includes, but is not limited to, at least one of water, a C₃ to C₇ ester, a C₃ to C₇ ketone, acetonitrile (ACN), acetone, methyl alcohol (MeOH, MeOH absolute), ethyl alcohol (EtOH, EtOH absolute), isopropyl alcohol (IPA), propyl alcohol (PrOH), butyl alcohol (BuOH, 2-BuOH, t-BuOH), amyl alcohol, methyl ethyl ketone (MEK), methyl isobutyl ketone (MIBK), dimethyl carbonate (DMC), diethyl carbonate (DEC), methyl acetate (MeOAc), ethyl acetate (EtOAc), butyl acetate (BuOAc), isobutyl acetate (iBuOAc), ethyl lactate, butyl lactate, methyl tertbutyl ether (MTBE), dibutylether, dichloromethane (CH₂Cl₂), toluene, petroleum ether 60-80, hexane, cyclohexane, heptane, propylene glycol, tetrahydrofuran (THF), and chlorobenzene. The amount of the solvent should be sufficient to dissolve the montelukast. One of ordinary skill in the art can easily determine the sufficient amount of the solvent. Preferably, the dissolving step further includes stirring the solution. Stirring can be achieved by any means including, but not limited to, mechanical and magnetic means The dissolving step may further include facilitative measures known to one skilled in the art. For example, the dissolving step may further include heating, filtering, and/or diluting the solution.

The process may further include adding an anti-solvent. Examples of anti-solvents include C₅ to C₁₂ hydrocarbons such as heptane and hexane. When the solvent is used with an anti-solvent, the combination is described as a ratio volume/volume.. Preferably, the anti-solvent is added dropwise to the solution until a precipitate begins to form.

The process may further include acidifying the solution, when the starting material is a salt. Acid may be added to reduce the pH of the montelukast solution, resulting in precipitation of montelukast acid. The pH may be adjusted by using aqueous acidic solutions including, but not limited to hydrochloric acid, sulfuric acid, formic acid, and acetic acid.

Preferably, the crystallization step is performed with stirring. The crystallization step can be performed at about 20°C to about 25°C ("room temperature" or "RT") or at an elevated temperature of at least about 40°C, preferably about 60°C. The crystallization step can be performed for about 1 hour to about 72 hours. The crystallization step may further include facilitative measures known to one skilled in the art. For example, the crystallization step may further include cooling the solution, heating the solution, or adding an agent to induce precipitation.

Recovering the crystalline form of montelukast acid can be performed by any means known in the art including, but not limited to, filtration, centrifugation, and decanting. Preferably, the crystalline form is recovered by filtration. The crystalline form may be recovered from any composition containing the crystalline form and the solvent(s) including, but not limited to, a suspension, solution, slurry, and emulsion.

The process may further include washing the crystalline form.

The process may further include drying the crystalline form. Drying can be performed under ambient or reduced pressure For example, drying can be performed under reduced pressure, preferably about 10-50 mm Hg, at a temperature of at least about 40°C, preferably about 50°C for about 1 to about 3 days

In another embodiment, the invention encompasses processes for crystallizing montelukast free acid Form I including the steps of crystallizing the crystalline form from a solution of montelukast in a solvent and recovering the crystalline form. The solution is prepared by dissolving montelukast in an organic solvent. Preferably, the montelukast starting material is montelukast free acid. Preferably, the organic solvent is selected from the group consisting of: water, ACN, acetone, methyl alcohol absolute, methyl alcohol, ethyl alcohol absolute, IPA, propyl alcohol, butyl alcohol, MEK, MIBK, DMC, DEC, methyl acetate, ethyl acetate, isobutyl acetate, ethyl lactate, butyl lactate, MTBE, methylene chloride, toluene, petroleum ether 60-80, hexane, cyclohexane, heptane, propylene glycol, a combination of THF and heptane (1:2), or mixtures thereof. Preferably, the dissolving step further includes stirring the solution. The process may further include adding an anti-solvent. Examples of anti-solvents include C₅ to C₁₂ hydrocarbons such as heptane and hexane. Preferably, the crystallization step further includes stirring the solution. The crystallization step can be performed at room temperature, or it may further include heating the solution to at least about 40°C, preferably about 60°C. Preferably, the crystallization step is performed for about 24 to about 72 hours. Preferably, the crystalline form is recovered by filtration. The process may further include washing the crystalline form, preferably with the solvent. The process may further include drying the crystalline form.

In another embodiment, the invention encompasses processes for crystallizing montelukast free acid Form II including the steps of crystallizing the crystalline form from a solution of montelukast in chlorobenzene, and recovering the crystalline form. The solution is prepared by dissolving montelukast in chlorobenzene. Preferably, the dissolving step further includes stirring the solution. Preferably, the montelukast starting material is montelukast free acid. Preferably, the crystallization step is performed at about room temperature. Preferably, the crystallization step is performed for at least about 24 hours. Preferably, the crystallization step further includes stirring the solution. Preferably, the crystalline form is recovered by filtration. The process may further include washing the crystalline form, preferably with chlorobenzene. The process may further include drying the crystalline form.

One embodiment of the invention encompasses an amorphous form montelukast free acid.

The amorphous form of a drug generally has enhanced solubility and bioavailability.

In one embodiment, the invention encompasses processes for preparing amorphous montelukast free acid including the steps of precipitating amorphous montelukast free acid from a solution of montelukast salt in water, and recovering the precipitate. The solution is prepared by dissolving montelukast in water. Preferably, the montelukast starting material is montelukast sodium salt The process further include acidifying the solution to precipitate montelukast free acid. Preferably, the acidification is performed by adding HCl. Preferably, the HCl is added dropwise to the solution until a precipitate begins to form. Preferably, the dissolving step further includes stirring the solution. Preferably, the precipitation step is performed at room temperature. Preferably, the precipitation step is performed for about 1 to about 72 hours. Preferably, the precipitation step further includes stirring the solution. Preferably, the precipitate is recovered by filtration. The process may further include washing the precipitate, preferably with water. The process may further include drying the precipitate.

Many processes of the present invention involve crystallization out of a particular solvent. The term "crystallization" as used herein includes the dissolution of the starting compound to obtain a clear solution, maintaining the solution for a period of time with or without cooling or other inducement. The dissolution can take place at ambient temperature. One skilled in the art would appreciate that the conditions concerning crystallization can be modified without affecting the form of the polymorph obtained. For example, when mixing montelukast in a solvent to form a solution, warming of the mixture may be necessary to completely dissolve the starting material. If warming does not clarify the mixture, the mixture may be diluted or filtered. To filter, the hot mixture may be passed through paper, glass fiber or other membrane material, or a clarifying agent such as celite. Depending upon the equipment used and the concentration and temperature of the solution, the filtration apparatus may need to be preheated to avoid premature crystallization.

The conditions may also be changed to induce precipitation. A preferred way of inducing precipitation is to reduce the solubility of the solvent. The solubility of the solvent may be reduced, for example, by cooling the solvent.

In one embodiment, an anti-solvent is added to a solution to decrease its solubility for a particular compound, thus resulting in precipitation. Another way of accelerating crystallization is by scratching the inner surface of the crystallization vessel with a glass rod. Other times, crystallization may occur spontaneously without any inducement. The present invention encompasses both embodiments where crystallization of a particular form of montelukast free acid occurs spontaneously or is induced/accelerated, unless if such inducement is critical.

As used herein, an anti-solvent is a liquid that when added to a solution of X in the solvent, induces precipitation of X. Precipitation of X is induced by the anti-solvent when addition of the anti-solvent causes X to precipitate from the solution more rapidly or to a greater extent than X precipitates from a solution containing an equal concentration of X in the same solvent when the solution is maintained under the same conditions for the same period of time but without adding the anti-solvent. Precipitation can be perceived visually as a clouding of the solution or formation of distinct particles of X suspended in the solution or collected at the bottom the vessel containing the solution.

The invention also provides processes for preparing montelukast sodium with high purity. In one embodiment, the process includes obtaining montelukast free acid as a solid and converting the montelukast free acid to montelukast sodium. A process for purifying montelukast sodium may include the steps of dissolving montelukast sodium to form montelukast free acid, crystallizing the free acid, and converting the free acid to montelukast sodium with high purity. The montelukast free acid can be crystallized by any of the embodiments of the present invention. The montelukast free acid can be converted to montelukast sodium by any means known in the art, for example, using sodium hydroxide (NaOH). For example, montelukast free acid can be converted to montelukast sodium by slurrying in a liquid in the presence of a sodium base. Preferably the liquid is selected from the group consisting of methanol (MeOH), ethanol (EtOH), butanol (BuOH), acetone, methyl isobutyl ketone (MIBK), isobutylacetate, heptane, isopropylether, toluene, acrylonitrile (ACN), dimethyl carbonate (DMC), and mixtures thereof. Most preferably the liquid is DMC. Preferably the sodium base is NaOH or sodium metoxide. In one embodiment, montelukast free acid is converted to montelukast sodium using dimethyl carbonate (DMC) and either NaOH or sodium tert-butoxide (t-BuONa).

One skilled in the art may also appreciate that the scope of the disclosure is not limited by the order of the additions in adding an anti-solvent. For example, a solution may be added to an anti-solvent or vice versa, though an embodiment may prefer one over the other. Usually crystallization is better when a solution is added to the anti-solvent, but operationally it is often more convenient to add the anti-solvent to the solution.

The starting material used for the processes of the present invention may be any crystalline or amorphous form of montelukast, including any salts, solvates, and hydrates. The montelukast starting material can be, for example, amorphous montelukast free acid, montelukast sodium, montelukast lithium, montelukast calcium, montelukast potassium, or montelukast dicyclohexylamine salt. With processes where montelukast goes into solution, the form of the starting material is of minimal relevance since any solid state structure is lost in solution. With suspension and drying processes, the starting material may sometimes make a difference, as one of skill in the art would appreciate.

One embodiment of the invention encompasses pharmaceutical compositions containing the crystalline forms of montelukast free acid of the invention and methods of treating respiratory diseases using the same.

Pharmaceutical compositions of the present invention contain crystalline montelukast such as one of those disclosed herein, or montelukast purely amorphous, optionally in mixture with other form(s) of montelukast. Montelukast that is crystallized by the processes of the present invention is ideal for pharmaceutical formulation. In addition to the active ingredient(s), the pharmaceutical compositions of the present invention may contain one or more excipients. Excipients are added to the composition for a variety of purposes.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel® ), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit® ), potassium chloride, powdered cellulose, sodium chloride, sorbitol, and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel® ), hydroxypropyl methyl cellulose (e.g. Methocel® ), liquid glucose, magnesium aluminum silicate, maltodextrin, methylcellulose, polymethacrylates, povidone (e.g. Kollidon® , Plasdone® ), pregelatinized starch, sodium alginate, and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol® , Primellose® ), colloidal silicon dioxide, croscarmellose sodium, crospovidone (e.g. Kollidon® , Polyplasdone® ), guar gum, magnesium aluminum silicate, methyl cellulose, microcrystalline cellulose, polacrilin potassium, powdered cellulose, pregelatinized starch, sodium alginate, sodium starch glycolate (e.g. Explotab® ), and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dyed using any pharmaceutically acceptable colorant to improve their appearance and/or facilitate patient identification of the product and unit dosage level.

In liquid pharmaceutical compositions of the present invention, montelukast and any other solid excipients are dissolved or suspended in a liquid carrier such as water, vegetable oil, alcohol, polyethylene glycol, propylene glycol, or glycerin

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, and xanthan gum.

Sweetening agents such as sorbitol, saccharin, sodium saccharin, sucrose, aspartame, fructose, mannitol, and invert sugar may be added to improve the taste.

Preservatives and chelating agents such as alcohol, sodium benzoate, butylated hydroxy toluene, butylated hydroxyanisole, and ethylenediamine tetraacetic acid may be added at levels safe for ingestion to improve storage stability.

According to the present invention, a liquid composition may also contain a buffer such as guconic acid, lactic acid, citric acid or acetic acid, sodium guconate, sodium lactate, sodium citrate, or sodium acetate. Selection of excipients and the amounts used may be readily determined by the formulation scientist based upon experience and consideration of standard procedures and reference works in the field.

The solid compositions of the present invention include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the present invention may contain any of the aforementioned blends and granulates that were described with reference to tableting, however, they are not subjected to a final tableting step.

Methods of administration of a pharmaceutical composition for treating respiratory diseases, especially asthma, encompassed by the invention are not specifically restricted, and can be administered in various preparations depending on the age, sex, and symptoms of the patient. For example, tablets, pills, solutions, suspensions, emulsions, granules, and capsules may be orally administered. Injection preparations may be administered individually or mixed with injection transfusions such as glucose solutions and amino acid solutions intravenously. If necessary, the injection preparations are administered singly intramuscularly, intracutaneously, subcutaneously, or intraperitoneally. Suppositories may be administered into the recturn.

The amount of montelukast free acid contained in a pharmaceutical composition for treating respiratory diseases, especially asthma, according to the present invention is not specifically restricted, however, the dose should be sufficient to treat, ameliorate, or reduce the symptoms associated with the respiratory disease. The dosage of a pharmaceutical composition for treating respiratory diseases according to the present invention will depend on the method of use, the age, sex, and condition of the patient. Typically, about 4 mg, 5 mg, or 10 mg of the montelukast free acid may be contained in an administration form unit.

Having described the invention, the invention is further illustrated by the following non-limiting examples.

### EXAMPLES

### Example 1: Crystallizing amorphous montelukast free acid

Montelukast sodium (50 g) was dissolved in water (750 mL) and stirred at room temperature to form a solution. Hydrochloric acid (1N HCl, 0.85 eq, 70 mL) was added dropwise until the solution reached a pH of 6 and a precipitate started to form. Then, the solution was stirred at room temperature for 1 hour. The precipitate was recovered by filtration, washed with water (15 mL), and dried under reduced pressure, 10-50 mm Hg, at 50°C for 32 hours to obtain amorphous montelukast acid (47.2 g, 97.9% yield). The results are summarized in Table 1, below.

**Table 1: Results of crystallizing amorphous montelukast free acid Volume of solvent is in mL, per gram of montelukast.**

| **Solvent** | **Vol. (1g/mL)** | **Temp. (°C)** | **Time (hrs)** | **XRD** | |
|---|---|---|---|---|---|
| | | | | **Sample wet/dry** | **Form** |
| Water | 15 | RT | 2 | d | Amorphous |

### Example 2: Crystallizing montelukast free acid

Amorphous montelukast free acid (1.5 g) was dissolved in a solvent and stirred until a precipitate formed. Some solutions were stirred at room temperature; others were heated to 60°C. The precipitate was recovered by filtration and washed with the solvent (5 mL) to obtain a wet sample. A portion of the wet sample was dried overnight at 50°C at 10-50 mm Hg to obtain a dry sample. The wet and dry samples were analyzed by X-ray diffraction. The results are summarized on Table 2. When the solvent was a combination of solvents, Table 2 describes the ratio of solvents by volume/volume.

**Table 2: Results of crystallizing montelukast free acid Volume of solvent is in mL per gram of montelukast.**

| **Solvent** | **Vol. (1g/mL)** | **Temp. (°C)** | **Time (hrs)** | **XRD** | |
|---|---|---|---|---|---|
| | | | | **Sample wet/dry** | **Form** |
| Water | 6 | RT | 24 | w | Amorphous |
| | | | | d | Amorphous |
| Water | 6 | RT | 72 | w | I + Amorphous |
| | | | | d | I |
| Water | 6 | 60°C | 24 | w | I |
| | | | | d | I |
| ACN | 8 | RT | 24 | w | I |
| | | | | d | I |
| Acetone | 4 | RT | 24 | w | I |
| | | | | d | I |
| Acetone | 4 | RT | 72 | w | I |
| | | | | d | I |
| MeOH abs. | 4 | RT | 24 | w | I |
| | | | | d | I |
| MeOH | 4 | RT | 72 | w | I |
| | | | | d | I |
| EtOH abs. | 4 | RT | 24 | w | I |
| | | | | d | I |
| IPA | 4 | RT | 24 | w | I |
| | | | | d | I |
| IPA | 4 | RT | 72 | w | I |
| | | | | d | I |
| PrOH | 4 | RT | 24 | w | I |
| | | | | d | I |
| BuOH | 4 | RT | 24 | w | I |
| | | | | d | I |
| BuOH | 4 | RT | 72 | w | I |
| | | | | d | I |
| MEK | 4 | RT | 24 | w | I |
| | | | | d | I |
| MEK | 4 | RT | 72 | w | I |
| | | | | d | I |
| MIBK | 4 | RT | 24 | w | I |
| | | | | d | I |
| MIBK | 4 | RT | 72 | w | I |
| | | | | d | I |
| DMC | 4 | RT | 24 | w | I |
| | | | | d | I |
| DMC | 4 | RT | 72 | w | I |
| | | | | d | I |
| DEC | 4 | RT | 24 | w | I |
| | | | | d | I |
| MeOAc | 4 | RT | 24 | w | I |
| | | | | d | I |
| EtOAc | 4 | RT | 24 | w | I |
| | | | | d | I |
| EtOAc | 4 | RT | 72 | w | I |
| | | | | d | I |
| iBuOAc | 4 | RT | 24 | w | I |
| | | | | d | I |
| iBuOAc | 4 | RT | 72 | w | I |
| | | | | d | I |
| Ethyl Lactate | 4 | RT | 24 | w | I |
| | | | | d | I |
| Butyl Lactate | 4 | RT | 24 | w | I |
| | | | | d | I |
| MTBE | 4 | RT | 24 | w | I |
| | | | | d | I |
| CH₂Cl₂ | 4 | RT | 24 | w | I |
| | | | | d | I |
| Toluene | 4 | RT | 24 | w | I |
| | | | | d | I |
| Toluene | 4 | RT | 72 | w | I |
| | | | | d | I |
| Petroleum Ether | 3 | RT | 24 | w | I |
| 60-80 | | | | d | I |
| Petroleum Ether | 3 | 60°C | 24 | w | I |
| 60-80 | | | | d | I |
| Hexane | 4 | RT | 24 | w | I |
| | | | | d | I |
| Hexane | 4 | 60°C | 24 | w | I |
| | | | | d | I |
| Cyclohexane | 6 | RT | 24 | w | I |
| | | | | d | I |
| Cyclohexane | 6 | 60°C | 24 | w | I |
| | | | | d | I |
| Heptane | 6 | RT | 24 | w | I |
| | | | | d | I |
| Heptane | 6 | 60°C | 24 | w | I |
| | | | | d | I |
| Propylene | 8 | RT | 24 | w | I |
| Glycol | | | | d | I |
| THF:Heptane | 12 | RT | 24 | w | I |
| (1:2) (vol/vol) | | | | d | I |
| Chlorobenzene | 4 | RT | 24 | w | II |

### Example 3: X-ray diffraction analysis

The crystal forms were identified using an ARL Applied Research Laboratory (SCINTAG) powder X-ray diffractometer model X'TRA equipped with a solid state detector. The crystal samples were analyzed using a round aluminum sample holder with zero background and copper radiation of 1.5418 Å.

**Table 3: X-ray diffraction peaks for crystalline forms of montelukast free acid Peaks are measured in degrees two-theta ± 0.2 degrees two-theta. Peaks in bold are the most characteristic peaks.**

| Form I | Form II |
|---|---|
| 6.5 | 9.1 |
| 10.0 | 9.4 |
| 13.1 | 10.3 |
| 15.5 | 10.8 |
| 17.6 | 16.0 |
| 18.3 | 16.5 |
| 20.4 | 19.0 |
| 24.6 | 18.7 |
| 26.3 | 20.6 |
| 27.8 | 22.7 |
| 28.8 | 23.2 |
| 31.7 | 23.6 |

### Example 4: Additional Solvents and Conditions for Crystallizing Montelukast Free Acid

The general procedure for montelukast acid crystallization is described below. The specific reaction conditions are shown in Table 4.

To a 100 mL flask equipped with a magnetic stirrer and a reflux condenser, montelukast free acid (1.5 g) and solvent (3.75 mL) were added. The mixture was heated and solvent was added to obtain a clear solution. After obtaining a clear solution, the mixture was cooled slowly to the indicated temperature.

**Table 4: Crystallization of Montelukast free acid**

| Solvent | Total Volume (mL/g MLK) | Heating Temp | Cooling Temp | Yield(%) |
|---|---|---|---|---|
| MeOAc | 8 | Reflux | RT | 60 |
| MeOAc | 8 | Reflux | 5°C | 74 |
| EtOAc | 5.33 | Reflux | RT | 76 |
| BuOAc | 3.66 | Reflux | RT | 80 |
| ACN | 20 | Reflux | RT | 80 |
| ACN | 94 | 60°C | RT | 80 |
| ACN | 20 | Reflux | RT | 86 |
| ACN:Acetone (7:8) | 10 | 60°C | RT | 52.6 |
| EtOH | 4 | Reflux | RT | 74 |
| EtOH | 4 | Reflux | 5°C | 82 |
| EtOH:ACN (1.3:10) | 7.5 | Reflux | RT | 80 |
| 2-BuOH | 2.66 | Reflux | RT | 80 |
| t-BuOH | 5.33 | Reflux | RT | 84 |
| Amyl-OH | 2.66 | Reflux | RT | 76.6 |
| DEC | 2.66 | 110°C | RT | 77 |
| MEK | 2.66 | Reflux | RT | 32 |
| MEK | 4 | 60°C | RT | 43 |
| Acetone then Water (2.6:1) | 6 | Reflux | 5°C | 80 |
| MIBK | 2.6 | 96°C | RT | 52 |
| DiBu-ether | 40 | 110°C | RT | 84 |
| MTBE | 50 | Reflux | RT | 33 |
| Toluene | 6.66 | Reflux | RT | 72 |

Having thus described the invention with reference to particular preferred embodiments and illustrative examples, those in the art can appreciate modifications to the invention as described and illustrated that do not depart from the spirit and scope of the invention as disclosed in the specification. The Examples are set forth to aid in understanding the invention but are not intended to, and should not be construed to, limit its scope in any way. The examples do not include detailed descriptions of conventional methods. Such methods are well known to those of ordinary skill in the art and are described in numerous publications. Polymorphism in Pharmaceutical Solids, Drugs and the Phamiaceutical Sciences, Volume 95 may be used for guidance. All references mentioned herein are incorporated in their entirety.

## Claims

1. A crystalline form of montelukast free acid (Form I) **characterized by** an XRD pattern with peaks at 6.5, 10.0, 13.1, 155, 17.6, and 18.3 degrees two-theta ± 0 2 degrees two-theta

2. The crystalline form of claim 1 further **characterized by** X-ray powder diffraction peaks at 20.4, 24.6, 26.3, 27.8, 28.8, and 31.7 degrees two-theta ± 0.2 degrees two-theta.

3. The crystalline form of claim 1, wherein the crystalline form has a powder XRD pattern substantially as depicted in Figure 1.

4. A process for preparing the crystalline montelukast free acid of claim 1 (Form I), comprising:
a) dissolving montelukast in at least one organic solvent to form a solution;
b) precipitating the crystalline montelukast free acid (Form I) from the solution; and
c) recovering the precipitated crystalline montelukast free acid (Form I).

5. The process of claim 4, wherein the montelukast of step a) is montelukast free acid.

6. The process of claim 4, wherein the solvent is selected from the group consisting of: water, acetonitrile, acetone, methyl alcohol absolute, methyl alcohol, ethyl alcohol absolute, isopropyl alcohol, propyl alcohol, butyl alcohol, methyl ethyl ketone, methyl isobutyl ketone, diethyl carbonate, methyl acetate, ethyl acetate, isobutyl acetate, ethyl lactate, butyl lactate, methyl tertbutyl ether, methylene chloride, toluene, petroleum ether, hexane, cyclohexane, heptane, propylene glycol, and a combination of tetrahydrofuran and heptane.

7. The process of claim 4, wherein the dissolving step includes stirring the solution.

8. The process of claim 4 further comprising heating the solution to at least about 40°C.

9. The process of claim 4 further comprising stirring the reaction mixture in step b)..

10. The process of claim 4, wherein the precipitating step is performed for about 24 to about 72 hours.

11. The process of claim 4 further comprising adding an anti-solvent to the solution to precipitate the crystalline form.

12. The process of claim 11, wherein the anti-solvent is a C₅ to C₁₂ hydrocarbon.

13. The process of claim 11, wherein the anti-solvent is heptane or hexane.
